# EUROPEAN PATENT SPECIFICATION

(11) **EP 1 977 727 B1**
(45) Date of publication and mention of the grant of the patent: **15.12.2010**
(21) Application number: 07007135.2
(22) Date of filing: 04.04.2007
(51) Int. Cl.: A61K 6/00, A61Q 11/00, A61K 49/00

(54) **Composition for dyeing dental plaque for evaluating dental caries activity**
Zusammensetzung zum Färben von Zahnbelag zur Beurteilung der Zahnkariesaktivität
Composition pour colorer la plaque dentaire afin d'évaluer l'activité des caries dentaires

(43) Date of publication of application: 08.10.2008
(73) Proprietor: GC Corporation, Itabashi-ku Tokyo 174-8585 (JP)
(72) Inventor: Walsh, Laurence J., Ferny Hills, QLD 4055 (AU); Ota, Ryoko, Itabashi-ku Tokyo 174-8585 (JP); Nagao, Sayaka, Itabashi-ku Tokyo 174-8585 (JP); Sato, Takuya, Itabashi-ku Tokyo 174-8585 (JP)
(74) Representative: Müller-Boré & Partner Patentanwälte

(56) References cited:
- JP-A- 2002 348 224
- US-A- 3 997 658
- US-A- 4 397 944
- US-A1- 2004 228 762

## Description

The present invention relates to a composition for dyeing dental plaque for evaluating dental caries activity, by which dental plaque can be dyed and identified and caries activity can be evaluated.

Dental plaque is a structured bacterial deposit including a mixed bacterial flora and an organic matrix derived from bacteria and from saliva. It is formed continuously on tooth surfaces, and causes both dental caries and a periodontal disease. It is important to remove deposits of dental plaque on a regular basis, to keep the inside of the oral cavity clean and the teeth healthy. However, since it is difficult with the naked eye to identify the deposits of dental plaque that have adhered on the surfaces of the teeth, various kinds of a composition for dyeing dental plaque have been conventionally used to certainly identify deposits of dental plaque on the teeth (for example, refer to Japanese Patent Application Laid Open No. 7(1995)-69852, 8(1996)-59513, 8(1996)-143477, 10(1998)-175835, 2002-138056, 2003-52725, 2004-151002, 2004-256504, 2005-139128, 2005-179188, 2005-325095, 2006-28152).

As a composition for dyeing dental plaque, for example, a colorant for food as a pigment for dyeing dental plaque is normally used. As a method of using the composition, the following method has been generally used, that is, a method comprising; including the composition in an oral cavity so as to dye dental plaque; rinsing out the composition for dyeing dental plaque if necessary; and observing the inside of the oral cavity so as to confirm the existence of the dyed dental plaque.

On the other hand, evaluation of dental caries activity has been widely carried out for preventing disease in an oral cavity. The evaluation of dental caries activity is carried out for predicting and determining the activity of dental caries, that is, for predicting and determining whether dental caries affecting the teeth is at an advanced state or not, or whether there is a possibility of dental caries starting in the future. This can be predicted by assessing whether the bacterial components of dental caries activity exists, whether or not the condition of dental caries is in a state of onset in the teeth.

As a composition used for evaluating the dental caries activity, in prior art an aqueous solution including a pH indicator and sugar has been generally used in an ex vivo fermentation test carried out at chairside, where the pH indicator gives a colored change within the specified range. As for the operation of the composition, the evaluation of dental caries activity is carried out by: contacting the dental plaque sample taken from the mouth with the aqueous solution so as to allow caries-related (fermenting) bacteria in dental plaque to metabolize the sugar; releasing acid from the dental plaque so as to decrease the pH of the aqueous solution; and changing the color of the aqueous solution when pH is decreased more than the discolored point of the pH indicator included in the aqueous solution (for example, refer to Japanese Patent Application Laid Open No. 50(1975)-1589, 54(1979)-47700, 56(1981)-96700, 56(1981)-120623, 57(1982)-13824, 59(1984)-99354, 2004-205210, 2002-348224).

However, the conventional composition for dyeing dental plaque described above can confirm only the dental plaque of the teeth, but does not evaluate dental caries activity. Further, as for the conventional composition used for evaluating dental caries activity, since the risk is determined by only the dental plaque taken from the teeth of a part in an oral cavity, there are problems that the error difference according to parts in which the dental plaque is taken out is large, and it cannot confirm which part of the teeth in the whole oral cavity has the highest-risk.

A dyeing dental plaque agent for detecting a white spot has been developed, where the agent can directly evaluate a part having the high carious risk by blending a pigment in which pH at the discolored point of a color tone is 4 to 7 (for example, refer to Japanese Patent Application Laid Open No. 2002-348224).

Document US 2004 1 228762 discloses a composition (and a kit) for evaluating dental caries, said composition comprising one pH indicator range of pH 3.5 to 8 and a sugar. This document does not mention the combination of a red an a blue pigment.

However, as for the dyeing dental plaque agent for detecting a white spot, the existence of the white spot (the evaluation of dental caries activity) must be determined by the change of the color tone of the pigment itself in which pH at the discolored point of a color tone is 4 to 7. Thus, when the dental plaque is thick, there is a problem that the accurate evaluation is difficult by assessing the color.

An objective of the present invention is to provide a composition for dyeing dental plaque for evaluating dental caries activity capable of simultaneously dyeing dental plaque and evaluating dental caries activity, and for evaluating the thick dental plaque more accurately than the conventional technique.

The earnest work was carried out in order to solve the above-described problems and, as a result of this, the followings were found out to complete the present invention.

That is, as for a composition for dyeing dental plaque including the conventional pigment for dyeing dental plaque and sugar so as to allow bacteria to metabolize the sugar. This is done by dental caries-related bacteria that existing in dental plaque, providing information on the risk in particular sites in the mouth. The following characteristics are used, that is, the deposits of dental plaques being thin and thick can be dyed by a red pigment but the dental plaque being thick can be dyed a blue pigment (refer to U. S. Patent No. 3997658). In this composition, the blue pigment and the red pigment are blended with sugar, where the blue pigment is dissolved in water and has a color tone not changing at pH 7 or less, and the red pigment is dissolved in water at pH more than 4.5 but not dissolved in water at the pH 4.5 or less and has a color tone not changing at pH 4.5 or less. Then, the comparatively thick dental plaque is dyed by the both the red pigment and the blue pigment when the pH is at a value before the bacteria metabolize sugar, but the red pigment is not dissolved in water at the portion in which the sugar is metabolized so as to be pH 4.5 or less. Thus, the red pigment can be washed and removed from the inside of an oral cavity so that the dental plaque can be made in the state that the dental plaque is not dyed by the red pigment. When using such a change of the dyeing state of the red pigment after and before metabolizing the sugar, accurate dyeing the dental plaque and accurate evaluation of the dental caries activity can be carried out even if the dental plaque is thick, by combining a purple dyeing state including the red and blue colors and a blue dyeing state in which the red color is removed by the change of pH.

That is, the present invention relates to a composition for dyeing dental plaque for evaluating dental caries activity, which includes: a blue pigment being dissolved in water and having a color tone not changing at pH 7 or less; a red pigment being dissolved in water at pH more than 4.5 but not dissolved in water at pH 4.5 or less and having a color tone not changing at pH of 4.5 or less; and a suitable substrate for bacterial fermentation which relates to dental caries, that is sucrose (table sugar). Other substrates could also be used in variations of this same test method, for example, fructose or glucose.

The composition for dyeing dental plaque for evaluating dental caries activity according to the present invention can simultaneously dye dental plaque and evaluate dental caries activity, and can evaluate the dental caries activity in the whole of the oral cavity. Thus, there is no error which could be caused by looking only in one site, since the test method uses a liquid provided to the whole mouth at one time, and does not rely on the clinician selecting one or two sites of interest. It can confirm which surfaces of the teeth in the oral cavity have the highest risk at that point of time. Further, even when dental plaque is thickly adhered on the teeth, the dental plaque can be evaluated without error by this technique, which overcomes the problem of the conventional method. So, the composition for dyeing dental plaque for evaluating dental caries activity according to the present invention is excellent.

A composition for dyeing dental plaque for evaluating dental caries activity according to the present invention, which includes: a blue pigment being dissolved in water and having a color tone not changing at pH 7 or less; a red pigment being dissolved in water at pH more than 4.5 but not dissolved in water at pH 4.5 or less and having a color tone not changing at the pH of 4.5 or less; and sugar.

As the blue pigment being dissolved in water and having a color tone not changing at pH 7 or less, a triphenylmethane-based pigment can be used, and more particularly, Blue No.1 (Brilliant Blue FCF), Green No.3 (Fast Green FCF) , and the like can be used. Those blue pigments can be used by combining them.

As the red pigment being dissolved in water at pH more than 4.5 but not dissolved in water at pH 4.5 or less and having a color tone not changing at pH 4.5 or less, a xanthene-based pigment can be used, and more particularly, Red No.3 (Erythrosine), Red No.104 (Phloxine), Red No.105 (Rose Bengal), Red No.106 (Acid Red) and the like can be used. Two or more kinds of those red pigments can be used by combining them.

With regards to the sugar, the fermentable mono saccharide and disaccharide sugars conventionally used for evaluating dental caries activity can be used without restriction. For example, glucose, sucrose, fructose, lactose, malt sugar, isomalto oligosaccharide, panose oligosaccharide, coupling sugar, isomerized sugar can be used.

As for the blending ratio of each component in the composition for dyeing dental plaque for evaluating dental caries activity according to the present invention, the content of sugar is preferably 1 to 50 weight parts to the total of 1 weight part of contents of the blue pigment and the red pigment, and the ratio of contents of the blue pigment and the red pigment is preferably 1:3 to 3:1. The reason for this is as follows. That is, if the content of sugar is less than 1 weight part to 1 weight part which is the total of contents of the blue pigment and the red pigment, the content of sugar is too low, so that when the sugar is metabolized by dental caries-related bacteria in dental plaque, the level of fermentation is too low to produce a dramatic visual change, and thus the dental caries activity cannot be accurately evaluated. Further, if the content of sugar is more than 50 weight parts, the ratios of water and alcohol are decreased, where the water and alcohol are added when the composition for dyeing dental plaque for evaluating dental caries activity according to the present invention is made into a liquid or paste state. In addition, very high levels of sugar would affect the viscosity of the preparation and make it unsuitable for clinical use, even though it would still serve as a substrate for plaque bacteria. Further, the reason that the ratio of contents of the blue pigment and the red pigment is preferably 1:3 to 3:1 is as follows. That is, if the ratio is out of this range, the content of either the blue pigment or the red pigment are too high. Thus, a part of the oral cavity having thin dental plaque which is dyed to red and a part having thick dental plaque which is dyed to purple will not be distinguished well and will be hard to classify. Furthermore, when the red pigment is deposited and flowed out at a part, where pH is less than 4 . 5 by bacteria in the dental plaque, there may be no difference between the color of the thin dental plaque part and the color of the thick dental plaque part, and the color of the thick dental plaque part may not be so clear that the dental caries activity cannot be accurately evaluated.

Such a composition for dyeing dental plaque for evaluating dental caries activity according to the present invention can be used in various kinds of modes, such as a liquid state, a paste state, a tablet state or the like, by the publicly known method.

When the composition is used as the liquid state, the liquid state composition can be produced by mixing the composition with water and/or alcohol to be prepared. When the composition is used as the paste state, the paste state composition can be produced by mixing the composition with water and/or alcohol and a thickening agent. Further, when the composition is used as the tablet state, the tablet state composition can be produced by preparing a thickening agent such as crystalline cellulose, carboxymethyl cellulose, hydroxypropyl cellulose or the like, which will be described below, as a binding and thickening agent.

The composition for dyeing dental plaque for evaluating dental caries activity according to the present invention is preferably used as the liquid state or the paste state from a point of usability. Especially, the paste state can increase adhesion to the teeth more than the liquid state, so that the dental plaque can be efficiently dyed by the paste state. As alcohol blended when the composition is used as the liquid or paste state, ethanol, glycerin, ethylene glycol, diethylene glycol, polyethylene glycol, propylene glycol, polypropylene glycol or the like can be used.

When the composition used as the paste state, the composition is preferably prepared so as to have the viscosity of the composition for dyeing dental plaque of 0.5 to 5 Pa·s at 25 degrees Celsius from the results of various tests relating to usability. As the thickening agent, the followings can be used, that is, a compound such as sodium arginine, alginic acid/propylene glycol ester, carboxymethyl cellulose, sodium carboxymethyl cellulose, calcium carboxymethyl cellulose, sodium starch glycolate, sodium starch phosphate, sodium starch phosphate ester, sodium polyacrylate, methylcellulose, crystalline cellulose, hydroxypropyl cellulose, polyvinyl pyrrolidone or the like, a natural material such as guar gum, carob bean gum, tara gum, tamarind seed gum, gumArabic, tragacanth gum, karaya gum, alginic acid, carrageenan, xanthan gum, gellan gum, curdlan, chitin, chitosan, chitosamin or the like, and inorganic materials such as calcium carbonate, calcium silicate, silica fine powders, amorphous hydrous silica, hydrophobic silica or the like.

The preferable viscosity range by the thickening agent described above is set to 0.5 to 5 Pa·s at 25 degree C from results of various tests relating to usability. That is, if the viscosity is less than 0.5 Pa·s, the effect intended by using the paste state is hardly obtained, and if the viscosity is more than 5 Pa·s, the adhesion to the teeth may be decreased. The blending ratio of the thickening agent to obtain the viscosity within this range can be achieved with various kinds of thickening agents. However, for example, as for sodium carboxymethyl cellulose used as the major thickening agent, the blending ration is about 0.2 to 12 weight parts, and as for methyl cellulose, the blending ratio is 5 to 65 weight parts. Accordingly, the proper blending ratio must be decided for every thickening agent employed.

The composition for dyeing dental plaque for evaluating dental caries activity according to the present invention can be properly constituted with an additive such as a cushioning material, a colorant, a preservative, an antiseptic agent, a fungicide, a pH regulator, a perfume, fluoride and the like, which have been conventionally used for compositions for use in the oral cavity.

### <Example>

The compositions of Examples were produced with the blending ratios shown in Table 1, and the following test was carried out to the compositions so as to evaluate those. Results were collectively shown in Table 1.

The tablet state compositions for dyeing dental plaque of Examples 7 and 8 were produced by: weighing and mixing each component; molding 10g of it using a single-shot tableting machine (the product name: 2B type, produced by Kikusui Seisakusyo Corporation) so as to have the hardness of 0.5 kg; and aging the molded tablet at 40 degree C and relative humidity of 75 % for 12 hours.
<Table 1>

**Table 1**

| Components | | Example 1 | Example 2 | Example 3 | Example 4 | Example 5 | Example 6 | Example 7 | Example 8 |
|---|---|---|---|---|---|---|---|---|---|
| States | | Paste-state | Paste-state | Paste-state | Paste-state | Paste-state | Liquid-stat, | Tablet-state | Tablet-state |
| | Glycerin | 20 | 20 | 15 | 20 | 20 | | | |
| | Ethanol | 5 | 5 | 5 | 5 | 5 | | | |
| | Silica dioxide (Aerosil A300) | 2,7 | 3 | 2 | | 2 | | | |
| | Guar gum | | | 0,5 | 1 | | 2 | | |
| | Polyethylene glycol | | | | | | | 8 | 10 |
| | Xanthan gum | | | | 1,5 | 1 | 2 | | 3 |
| | Sodium carboxymethyl cellulose (Serogen F-SA) | | | | | 1 | | 7 | |
| | Sodium carboxymethyl cellulose (Serogen F-SG) | 1,8 | 1,5 | 2 | 2 | | 2 | | 10 |
| | Crystalline cellulose | | | | | | | 26 | 35 |
| Sugars | Sucrose | 10 | | 15 | 5 | 5 | | 3,7 | |
| | Glucose | | 10 | | | 5 | 20 | | 25 |
| | Lactose | | | | | | | 32 | |
| Red colorant | Red 105 for food | 0,7 | | | 0,7 | 1 | 0,7 | 0,3 | |
| | Red 3 for food | | 0,7 | 0,7 | | | | | 0,2 |
| Blue colorant | Blue 1 for food | 0,6 | | 0,6 | | 1 | 0,6 | 0,5 | 0,2 |
| | Green 3 for food | | 0,6 | | 0,6 | | | | |
| Additive | Ethyl para-oxybenzoate | 0,1 | 0,1 | 0,1 | 0,1 | 0,1 | 0,1 | | |
| | Magnesium stearate | | | | | | | 4,5 | 16,6 |
| | Xylitol | | | | | | | 18 | |
| | Purified water | 59,1 | 59,1 | 59,1 | 64,1 | 58,9 | 72,6 | | |

### <Evaluation of dental caries activity>

The compositions for dyeing dental plaque for evaluating dental caries activity of Examples 1 to 5 were applied on the whole teeth in an oral cavity of subjects with a brush. Then, after passing about 5 minutes, they washed their mouth, and the oral cavities of the subjects were observed. Then, it was confirmed that the red pigment was deposited to flow out at the part in which pH was 4.5 or less by bacteria in the dental plaque, so that it could be confirmed that the part in which the color of the thick dental plaque part was changed from purple to blue. That is, it was found out that the dental caries activity of the part which was discolored to blue in the dental plaque part was high.

Further, the compositions for dyeing dental plaque for evaluating dental caries activity of Examples 6 to 8 were included as to spread those to the whole oral cavities of the subjects, and breathed out after 15 seconds. After that, they washed their mouth after passing about 5 minutes, and oral cavities of the subjects were observed. Then, it was confirmed that the red pigment was deposited to flow out at the part in which the pH was 4.5 or less by bacteria in the dental plaque, so that it could be confirmed that the part in which the color of the thick dental plaque part was changed from purple to blue. That is, it was found out that the dental caries activity of the part which was discolored to blue in the dental plaque part was high.

## Claims

1. A composition for dyeing dental plaque for evaluation dental caries activity comprising:
a blue pigment being dissolved in water and having a color tone not changing at pH 7 or less;
a red pigment being dissolved in water at pH more than 4.5 but not dissolved in water at pH 4.5 or less, and having a color tone not changing at pH of 4.5 or less; and
sugar.

2. The composition for dyeing dental plaque for evaluation dental caries activity according to claim 1,
wherein the blue pigment comprises one or more kinds of Blue No. 1 (Brilliant Blue FCF), and Green No. 3 (Fast Green FCF), and the red pigment comprises one or more kinds of Red No.3 (Erythrosine), Red No.104 (Phloxine), Red No.105 (Rose Bengal), and Red No.106 (Acid Red).

3. The composition for dyeing dental plaque for evaluation dental caries activity according to claim 1 or 2,
wherein the content of sugar is 1 to 50 weight parts to the total of 1 weight part of contents of the blue pigment and the red pigment, and ratio of contents of the blue pigment and the red pigment is 1:3 to 3:1.

## Patentansprüche

1. Zusammensetzung zum Färben von Zahnbelag zur Beurteilung von Zahnkariesaktivität, umfassend:
ein blaues Pigment, das in Wasser gelöst wird und einen Farbton aufweist, der sich bei pH 7 oder weniger nicht ändert;
ein rotes Pigment, das in Wasser bei einem pH von mehr als 4,5 gelöst wird, aber in Wasser bei einem pH von 4,5 oder weniger nicht gelöst wird, und einen Farbton aufweist, der sich bei einem pH von 4,5 oder weniger nicht ändert; und
Zucker.

2. Zusammensetzung zum Färben von Zahnbelag zur Beurteilung von Zahnkariesaktivität nach Anspruch 1,
wobei das blaue Pigment eine oder mehrere Arten von Blau Nr. 1 (Brillantblau FCF) und Grün Nr. 3 (Fast Green FCF) umfasst, und das rote Pigment eine oder mehrere Arten von Rot Nr. 3 (Erythrosin), Rot Nr. 104 (Phloxin), Rot Nr. 105 (Rose Bengal) und Rot Nr. 106 (Säurerot) umfasst.

3. Zusammensetzung zum Färben von Zahnbelag zur Beurteilung von Zahnkariesaktivität nach Anspruch 1 oder 2,
wobei der Gehalt an Zucker 1 bis 50 Gewichtsteile zu der Gesamtheit von 1 Gewichtsteil der Gehalte des blauen Pigments und des roten Pigments beträgt, und wobei das Gehalteverhältnis des blauen Pigments und des roten Pigments 1:3 bis 3:1 beträgt.

## Revendications

1. Une composition pour colorer la plaque dentaire afin d'évaluer l'activité des caries comprenant :
un pigment bleu dissout dans de l'eau et ayant un ton de couleur ne changeant pas à un pH de 7 ou moins ;
un pigment rouge dissout dans de l'eau à un pH supérieur à 4,5, mais non dissout dans de l'eau à un pH de 4,5 ou moins, et ayant un ton de couleur ne changeant pas à un pH de 4,5 ou moins ; et
du sucre.

2. La composition pour colorer la plaque dentaire afin d'évaluer l'activité des caries selon la revendication 1,
dans laquelle le pigment bleu comprend un ou plusieurs types de Bleu n°1 (bleu brillant FCF), et Vert n°3 (vert solide FCF), et le pigment rouge comprend un ou plusieurs types de Rouge n°3 (érythrosine), de Rouge n°104 (phloxine), de Rouge n°105 (rose Bengale), et de Rouge n° 106 (rouge acide).

3. La composition pour colorer la plaque dentaire afin d'évaluer l'activité des caries selon la revendication 1 ou 2,
dans laquelle la teneur en sucre est de 1 à 50 parts en poids par rapport au total de 1 part en poids des teneurs du pigment bleu et du pigment rouge, et le rapport des teneurs du pigment bleu et du pigment rouge est de 1:3 à 3:1.
